# EUROPÄISCHE PATENTSCHRIFT

(11) **EP 0 495 214 B1**
(45) Veröffentlichungstag und Bekanntmachung des Hinweises auf die Patenterteilung: **10.08.1994**
(21) Anmeldenummer: 91121596.0
(22) Anmeldetag: 17.12.1991
(51) Int. Cl.: A61M 5/32

(54) **Kanüle**
Hollow needle
Canule

(30) Priorität: 17.01.1991 DE 4101231
(43) Veröffentlichungstag der Anmeldung: 22.07.1992
(73) Patentinhaber: Fresenius AG, 61350 Bad Homburg (DE)
(72) Erfinder: Zimmermann, Michael, W-6690 St. Wendel (DE)
(74) Vertreter: Fuchs Mehler Weiss

(56) Entgegenhaltungen:
- EP-A- 0 301 246
- DE-A- 2 230 632
- US-A- 4 383 530

## Beschreibung

Die Erfindung betrifft eine Kanüle nach dem Oberbegriff des Anspruches 1.

Übliche Punktionskanülen weisen als Einstechteil einen mehr oder weniger stark geneigten keilförmigen Anschliff des Rohrendes auf, was sich in der Draufsicht in einer ovalen, mit der Rohrbohrung fluchtenden Lumenöffnung darstellt, wobei die Lumenöffnungshinterkante eine Schneide bildet. Die Ränder des unteren Bereiches des Einstechteiles sind üblicherweise zu einer Spitze geschliffen.

Bei der Punktion führt eine derartige Anformung des Einstechteiles dazu, daß die scharfe hintere Schneide der Lumenöffnung in diese zwangsläufig hineindrängendes Material der zu durchstechenden Fläche ausstanzt, was zur Folge hat, daß das Punktionsloch vergrößert wird.

Dies ist einerseits bei Gewebepunktion aufgrund der Traumatisierung nachteilig und zum anderen erweist sich der Ausstanzeffekt als äußerst unerwünscht, wenn derartige Kanülen zum Punktieren von implantierbaren Port-Kathetern benutzt werden. Derartige Systeme bestehen in der Regel aus einer implantierten Kapsel, die einen Hohlraum zur Entnahme oder Applikation einer Flüssigkeit wie Blut, Blutkomponenten oder eines Medikamentes aufweist. Die Kapsel ist mit einem Katheter verbunden, der in ein Gefäß oder sonstigen Wirkort mündet. Die zur Haut des Patienten hingerichtete Wand der implantierten Kapsel besteht aus einer punktierbaren Elastomer-Membran, die mit der Kanüle durch die Haut durchstochen wird.

Das Einstechen einer Kanüle in die Membran der Kapsel verursacht Undichtigkeiten dadurch, daß die Schneide der Lumenöffnungshinterkante Elastomer-Material ausstanzt, wodurch Löcher entstehen, die sich nicht mehr über die Rückstellkraft des Materiales von selbst schließen. Dies führt dazu, daß die implantierte Kapsel schon nach wenigen Einstichen Leckagen aufweist. Weiterhin können ausgestanzte Elastomer-Partikel in den Patienten geschwemmt werden oder den Katheter verstopfen.

Aufgrund dieser Problematik weist eine dem Oberbegriff des Anspruches 1 entsprechende aus der EP-A-0 301 246 bekannte Kanüle ein Kanülenrohr und ein Einstechteil auf, das eine Lumenöffnung aufweist. Das Kanülenrohr weist einen Endabschnitt auf und das Einstechteil ist ein Teil eines hakenförmigen Bogenabschnittes, der mit dem Abschnitt verbunden ist, wobei der Lumenöffnung eine hintere innere Schneide und der vordere Einstechteil zugeordnet sind, der eine lanzettförmige Spitze mit Facettenschliff aufweist. Die Lumenöffnung ist hierbei in einer konkav ausgeformten Seite des Bogenabschnittes angeordnet und weist eine dem Endabschnitt benachbarte einwärts in den Bogenabschnitt gerichtete Hinterkante auf. Auch bei dieser Kanüle ist jedoch eine weitere Verminderung des Keileffektes wünschenswert.

Eine andere Ausführung einer Huberkanüle ist aus der US-A-2, 409,979 bekannt. Die beschriebene Ausführungsform weist ein gerades Kanülenrohr auf, das in eine seitwärts gerichtete Lumenöffnung mündet, die derart ausgestaltet ist, daß die der Lumenöffnung gegenüberliegende Kanülenwandseite schräg in Richtung der Achse gekrümmt ist. Die die Lumenöffnung umgebende Wandung ist eben zur Kanülenrohraußenfläche geschliffen und bildet am hinteren Rand eine Schneide.

Aufgrund der Schrägfläche des Kanülenrohres zeigt auch diese Ausführungsform einen Keileffekt bei der Punktion einer künstlichen oder natürlichen Wandung, so daß das beim Vorschieben der Kanüle in die Lumenöffnung drängende Material, begünstigt durch die kaum einwärts gerichtete Schneide, abgehobelt oder durch Quetschung beschädigt wird.
Es ist eine weitere Kanüle bekannt, bei der es sich um eine Nadel handelt, deren vorderes Einstechteil hakenförmig ausgebildet ist, wodurch bei der Punktion z.B. einer Vene die Verletzungsgefahr der gegenüberliegenden Gefäßwand vermieden werden soll.
Die vordere, runde Kante des Einstechteiles liegt in der Flucht der Außenwand des Kanülenrohres. Sie zeigt keinen flachen Anschliff, während der hintere Rand der Lumenöffnung zur Bildung einer Schneide flach angeschliffen ist. Die der Lumenöffnung gegenüberliegende Rohrseite bildet eine stark gekrümmte Fläche, die als Gleitfläche wirksam ist. Diese Anformung erzwingt bei der Punktion eine winkelige Anstellung, wobei der Punktionsverlauf einer Stichbahn entsprechend dem Krümmungsradius der Gleitfläche folgt. Bei diesem Vorgang wird zwangsläufig Material der zu durchstehenden Fläche in die Lumenöffnung gedrückt, das von der flachen hinteren Schneide ausgestanzt wird und so das Punktionsloch ebenfalls vergrößert.
Schließlich sind aus der GB-A-2 073 026 und der US-A-2 717 599 weitere Kanülen bekannt, die im wesentlichen jedoch die gleichen Probleme wie die zwei erläuterten Kanülen aufweisen.
Es ist demgegenüber Aufgabe der vorliegenden Erfindung, eine Kanüle der im Oberbegriff des Anspruches 1 angegebenen Art so auszubilden, daß bei der Punktion von natürlichen oder künstlichen Wandungen Ausstanzungen vermieden werden.
Die Lösung dieser Aufgabe erfolgt durch die Merkmale des Anspruches 1.
Dadurch wird erreicht, daß das Kanülenrohr zum einstechenden Teil hin seitlich abgeknickt und im weiteren Verlauf hakenförmig ausgebildet ist und eine in die konkave Ausformung des hakenförmig verlaufenden Rohres tangential eingeschliffene Lumenöffnung aufweist, die an ihrer Hinterkante eine, durch die Anformung des Rohres bedingte, stark einwärts gerichtete Schneide ausbildet. Bevorzugterweise bilden die Wandungen der Lumenöffnung eine lanzettförmige Spitze mit Facettenschliff, wobei die Spitze des Einstechteiles mit der gedachten Achsenlinie des ansonsten geraden Kanülenrohres fluchtet.

Mit der erfindungsgemäßen Kanüle ergibt sich ein verringerter Stanzeffekt bei der Punktion künstlicher oder natürlicher Wandungen, da die stark einwärts gerichtete Hinterkante der Lumenöffnung mit der gegenüberliegenden, durch die Hakenform auswärts gebogene, Kanülenwand derart zusammenwirkt, daß der Stichkanal durch den Rücken aufgeweitet wird und die Kanüle aufgrund der einwärts gerichteten Schlifföffnungshinterkante ohne Hobeleffekt durch die Wandung gleitet. Die mit der gedachten Rohrachsenlinie vorzugsweise fluchtende Kanülenspitze verbessert die Verminderung des Keileffektes weiterhin und verringert vorzugsweise die Gefahr des Einpressens von Material in die Lumenöffnung, was im Zusammenwirken mit der einwärts gerichteten Hinterkante der Lumenöffnung eine besonders vorteilhafte stanz- oder hobelfreie Punktion zusätzlich begünstigt.

Somit eignet sich die erfindungsgemäße Kanüle für die atraumatische Punktion von natürlichen Geweben oder künstlichen Membranen insbesondere dann, wenn die Kanülen aufgrund benötigter Flußvolumina einen großen Innendurchmesser aufweisen.

Weitere Einzelheiten, Merkmale und Vorteile der Erfindung ergeben sich aus nachfolgender Beschreibung von Ausführungsbeispielen anhand der Zeichnung.

Es zeigt:
- Fig. 1: eine Ausführungsform einer erfindungsgemäßen Kanüle in Seitenansicht,
- Fig. 2: die Kanüle gemäß Fig. 1 in Draufsicht, und
- Fig. 3: eine Bemaßungstabelle für eine Kanüle gemäß den grundlegenden Konstruktionsprinzipien der Fig. 1 und 2, wobei in Fig. 3 die Bemessungsparameter ferner den einzelnen Teilen der Kanüle zugeordnet sind.

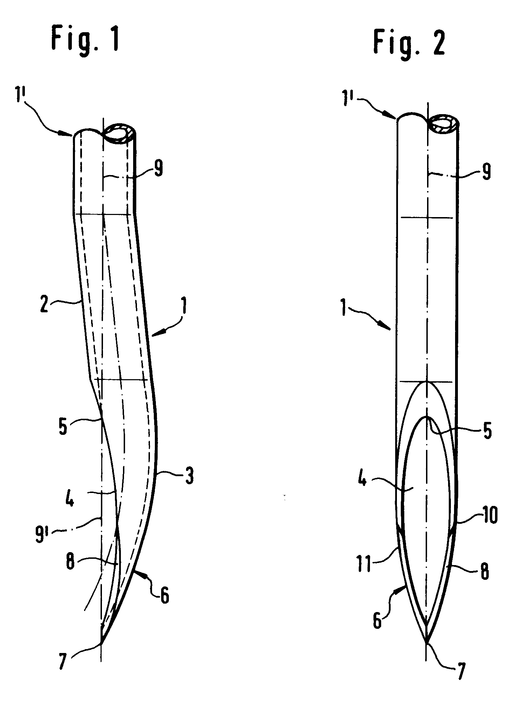

In den Fig. 1 und 2 ist eine Ausführungsform einer erfindungsgemäßen Kanüle 1 dargestellt, die ein gerades, vorzugsweise starres Kanülenrohr 1′ mit einer gestrichelt dargestellten Durchgangsausnehmung aufweist.

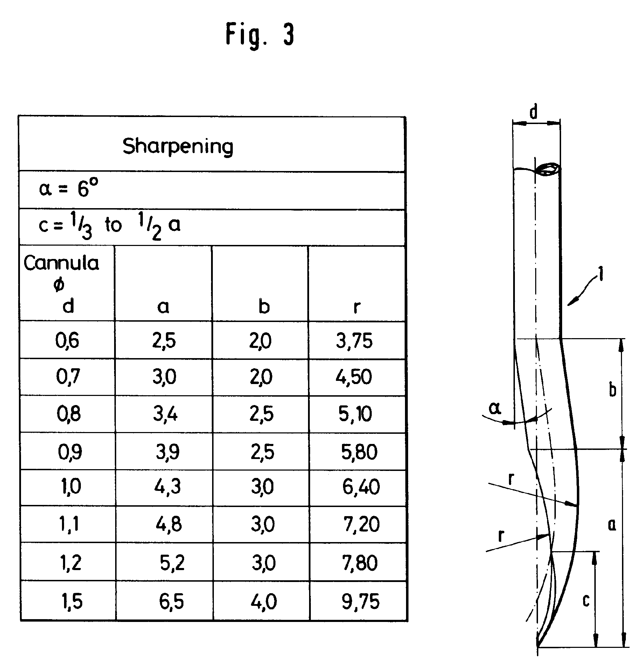

Die Kanüle 1 weist ferner einen Endabschnitt 2 auf, der sich an das Kanülenrohr 1′ anschließt. Fig. 1 verdeutlicht hierbei, daß der Endabschnitt 2 geradlinig wie das Kanülenrohr 1′ ausgebildet ist, jedoch bezüglich des Kanülenrohres 1′ seitlich abgeknickt ist, was bedeutet, daß der Endabschnitt einen Winkel mit der Kanülenrohr-Längsachse 9 einschließt, der in Fig. 3 als Winkel verdeutlicht ist.

Die Durchgangsausnehmung des Kanülenrohres 1′ setzt sich im Endabschnitt 2 fort und endet in einem Bogenabschnitt 3, der sich an den Endabschnitt 2 anschließt.

Der Bogenabschnitt 3 weist gemäß Fig. 1 zwei gerundete Längsränder mit dem Radius r auf, die aus Fig. 3 ersichtlich sind und deren Bemessung in der Tabelle der Fig. 3 in Abhängigkeit von unterschiedlichen Kanülendurchmessern angegeben sind.

Der Bogenabschnitt 3 weist ferner an seinem freien Ende ein Einstechteil 6 auf, das das vorderste Ende der Kanüle 1 bildet und in dem eine im Bogenabschnitt angeordnete Lumenöffnung 4 ausläuft. Fig. 2 verdeutlicht hierbei, daß die Lumenöffnung 4 linsenförmig ausgebildet ist, jedoch im Bereich einer Spitze 7 des Einstechteiles 6 in einer Öffnungsspitze ausläuft, während das der Öffenungsspitze gegenüberliegende Ende gerundet ist.

Die Fig. 1 und 2 verdeutlichen, daß das Einstechteil 6 ein Teil des hakenförmigen Bogenabschnittes 3 ist, der mit dem Endabschnitt 2 verbunden ist.

Die Lumenöffnung 4 ist in der konkaven Seite des Bogenabschnittes 3 angeordnet und weist eine dem Endabschnitt 2 benachbarte einwärts in den Bogenabschnitt 3 gerichtete Hinterkante 5 auf. Die in die konkave Ausformung des Bogenabschnittes 3 tangential eingeschliffene Lumenöffnung 4 bildet an ihrer Hinterkante 5 eine durch die Anformung des Endabschnittes 2 bedingte stark einwärts gerichtete Schneide, die maßgeblich zu den eingangs erläuterten Vorteilen der erfindungsgemäßen Kanüle beiträgt.

Bei der in den Fig. 1 und 2 dargestellten bevorzugten Ausführungsform der erfindungsgemäßen Kanüle 1 weist das Einstechteil 6 eine lanzettförmige Spitze 7 mit einem Facettenschliff 8 auf. Fig. 1 verdeutlicht hierbei, daß die Spitze 7 auf einer gedachten Kanülenrohr-Längslinie 9′ liegt bzw. mit dieser fluchtet, wobei die Längslinie 9′ die Verlängerung der Kanülenrohr-Längsachse 9 darstellt.

Fig. 2 zeigt, daß die lanzettförmige Spitze der dargestellten Ausführungsform der Kanüle 1 von Wandungen 10,11 der Lumenöffnung 4 gebildet wird.

In Fig. 3 ist eine Tabelle wiedergegeben, die in Abhängigkeit vom Kanülendurchmesser d beispielhaft einige bevorzugte Bemaßungen für die Konstruktionsparameter a (Länge des Bogenabschnittes 3), b (Länge des Endabschnittes 2), c (Länge des Einstechteiles 6), (Winkel zwischen dem Kanülenrohr 1′ und dem Endabschnitt 2 sowie r (Krümmungsradius der Längsränder des Bogenabschnittes 3) wiedergibt.

Die aus der Tabelle der Fig. 3 entnehmbaren Bemaßungen stellen jedoch nur Beispiele für Kanülen mit Durchmessern d von 0,6 bis 1,5 cm dar, bei denen sich dann die in der Tabelle enthaltenen bevorzugten Maße für die zuvor genannten Konstruktionsparameter ergeben. Andere Kanülendurchmesser sind jedoch ebenfalls mit den entsprechenden, besonders bevorzugten Konstruktionsparametern denkbar.

Aufgrund der zuvor beschriebenen Ausbildung der erfindungsgemäßen Kanüle 1 ist diese zur Punktion von natürlichen und künstlichen Wandungen besonders geeignet, da Ausstanzungen des zu perforierenden Materiales vermieden werden.

## Patentansprüche

1. Kanüle (1)
- mit einem Kanülenrohr (1′); und
- mit einem Einstechteil (6), das eine Lumenöffnung (4) aufweist und ein Teil eines hakenförmigen Bogenabschnitts (3) ist, der mit einem Endabschnitt (2) des Kanülenrohrs (1′) verbunden ist;
- wobei die Lumenöffnung (4) in einer konkav ausgeformten Seite des Bogenabschnittes (3) angeordnet ist und eine dem Endabschnitt (2) benachbarte einwärts in den Bogenabschnitt (3) gerichtete Hinterkante (5) aufweist,
dadurch gekennzeichnet,
- daß der Endabschnitt (2) des Kanülenrohrs (1′) in seitenansicht auf das Kanülenrohr (1′) gesehen, in einem spitzen Winkel (α) zur Kanülenrohr-Längsachse (9) angeordnet ist,
- daß die Spitze (7) auf einer gedachten Kanülenrohr-Längslinie (9′) liegt, die die Verlängerung der Kanülenrohr-Längsachse (9) ist, und
- daß der seitlich abgewinkelte Endabschnitt (2) gerade ist.

2. Kanüle nach Anspruch 1, dadurch gekennzeichnet, daß das Einstechteil (6) eine lanzettförmige Spitze (7) mit Facettenschliff (8) aufweist.

3. Kanüle nach Anspruch 1 oder 2, dadurch gekennzeichnet, daß der spitze Winkel (α) 6 Grad beträgt.

4. Kanüle nach einem der Ansprüche 1 bis 3, dadurch gekennzeichnet, daß die Länge (c) des Einstechteils (6) 1/3 bis 1/2 der Länge (a) des Bogenabschnittes (3) beträgt.

5. Kanüle nach einem der Anspräche 1 bis 4, dadurch gekennzeichnet, daß das Kanülenrohr (1′) gerade ist.

6. Kanüle nach einem der Ansprüche 1 bis 4, dadurch gekennzeichnet, daß das Kanülenrohr (1′) im Winkel von 90° gebogen ist.

7. Kanüle nach einem der Ansprüche 1 bis 6, dadurch gekennzeichnet, daß das Kanülenrohr (1′) starr ausgebildet ist.

## Claims

1. A cannula (1) comprising
- a cannula tube (1′); and
- a piercing part (6) having a lumen opening (4) and being part of a hook-shaped curved section (3) connected with an end section (2) of the cannula tube (1′);
- the lumen opening (4) being disposed in a concave side of the curved section (3) and having, adjacent to the end section (2), a rear edge (5) directed inwardly into the curved section (3),
characterized in
- that said end section (2) of said cannula tube (1′) seen in side view onto the cannula tube (1′) is disposed at an acute angle (α) to the longitudinal axis of the cannula (9),
- that the point (7) is on an imagined longitudinal axis of the cannula (9′) which is the extension of the longitudinal axis of the cannula (9), and
- that the laterally bent end section (2) is straight.

2. The cannula according to claim 1, characterized in that the piercing part (6) has a lancet-shaped point (7) with a beveled ground surface (8).

3. The cannula according to claim 1 or 2, characterized in that the acute angle (α) equals 6 degrees.

4. The cannula according to any one of claims 1 to 3, characterized in that the piercing part has a length (c) which equals 1/3 to 1/2 the length (a) of the curved section.

5. The cannula according to any one of claims 1 to 4, characterized in that the cannula tube (1′) is straight.

6. The cannula according to any one of claims 1 to 4, characterized in that the cannula tube (1′) is bent at an angle of 90°.

7. The cannula according to any one of claims 1 to 6, characterized in that the cannula tube (1′) is rigid.

## Revendications

1. Canule (1) comportant
- un tube de canule (1′) et
- une partie perforatrice (6) qui est pourvue d'une ouverture (4) de lumière et fait partie d'un segment arqué (3) en forme de crochet qui se raccorde à un segment d'extrémité (2) du tube (1′) de canule; l'ouverture (4) de la lumière étant disposée sur un côté de forme concave du segment arqué (3) et présentant une arête postérieure (5) voisine du segment d'extrémité (2) qui est tournée vers l'intérieur du segment arqué (3),
caractérisée par le fait que
- le segment d'extrémité (2) du tube (1′) de canule en vue de côté par rapport au tube (1′) de canule forme un angle (α) aigu avec l'axe longitudinal (9) du tube de canule,
- par le fait que la pointe (7) est située sur une ligne longitudinale (9′) imaginaire du tube de canule qui est le prolongement de l'axe longitudinal (9) du tube de canule, et
- par le fait que le segment d'extrémité (2) qui forme un angle latéralement est rectiligne.

2. Canule selon la revendication 1, caractérisée par le fait que la partie perforatrice (6) comporte une pointe (7) on forme de lancette polie de manière à former des facettes (8).

3. Canule selon la revendication 1 ou 2, caractérisée par le fait que l'angle aigu (α) est de 6 degrés.

4. Canule selon l'une des revendications 1 à 3, caractérisée par le fait que la longueur (c) de la partie perforatrice (6) représente 1/3 à 1/2 de la longueur (a) du segment arqué (3).

5. Canule selon l'une des revendications 1 à 4, caractérisée par le fait que le tube (1′) de canule est rectiligne.

6. Canule selon l'une des revendications 1 à 4, caractérisée par le fait que le tube (1′) de canule est coudé suivant un angle de 90 degrés.

7. Canule selon l'une des revendications 1 à 6, caractérisée par le fait que le tube (1′) de canule est rigide.
